(19) Europäisches Patentamt
European Patent Office
Office européen des brevets

(11) EP 3 395 838 A1

(12) # EUROPEAN PATENT APPLICATION
published in accordance with Art. 153(4) EPC

(43) Date of publication:
**31.10.2018 Bulletin 2018/44**

(21) Application number: **16879161.4**

(22) Date of filing: **04.11.2016**

(51) Int Cl.:
***C08B 15/02*** (2006.01) ***C07C 67/62*** (2006.01)
***C07C 69/80*** (2006.01) ***B01J 19/00*** (2006.01)

(86) International application number:
**PCT/KR2016/012621**

(87) International publication number:
**WO 2017/111294 (29.06.2017 Gazette 2017/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **24.12.2015 KR 20150186794**

(71) Applicant: **LOTTE Fine Chemical Co., Ltd.**
**Nam-gu, Ulsan 44714 (KR)**

(72) Inventors:
- **AHN, Jae Hoon**
  **Seoul 08259 (KR)**
- **SON, Jin Ryul**
  **Incheon 21995 (KR)**
- **BAE, Myung Hoon**
  **Seongnam-si**
  **Gyeonggi-do 13457 (KR)**

(74) Representative: **Michalski Hüttermann & Partner Patentanwälte mbB**
**Speditionstraße 21**
**40221 Düsseldorf (DE)**

(54) **HYDROXYPROPYL METHYLCELLULOSE PHTHALATE PARTICLES, AND PREPARATION METHOD THEREFOR**

(57) The present invention relates to hydroxypropyl methylcellulose phthalate (HPMCP) particles having a porous particle shape and a method of preparing the same. The preparation method includes (1) preparing an HPMCP reaction solution; (2) adding water to a stirring tank including a stirring device such that the stirring device is immersed therein; (3) dropping the HPMCP reaction solution into the stirring tank; and (4) adding the dropped HPMCP reaction solution to water contained in the stirring tank, in which the stirring device is operating, to be granulated while moving along the flow of the water. In accordance with the present invention, the HPMCP reaction solution is added to water contained in the stirring tank, in which the stirring device is operating, to granulate the HPMCP, whereby porous HPMCP particles can be prepared. Accordingly, the solubility of the particles can be improved.

【Fig. 1】

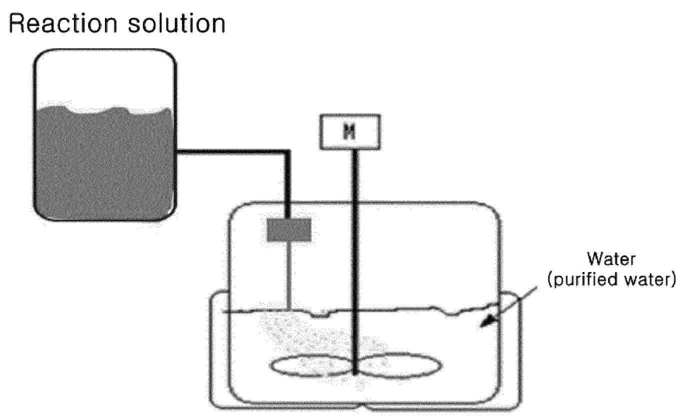




Printed by Jouve, 75001 PARIS (FR)

**Description**

[Technical Field]

The present invention relates to hydroxypropyl methylcellulose phthalate particles and a method of preparing the same, and more particularly to hydroxypropyl methylcellulose phthalate particles having a porous particle shape and, accordingly, having improved solubility in a solvent, and a method of preparing the same.

[Background Art]

Hydroxypropyl methylcellulose phthalate (HPMCP), as a vegetable polymer, is a derivative of cellulose, which is the main constituent of plant cell walls. Since HPMCP does not dissolve for 2 to 4 hours or longer under the pH condition of gastric juice (near pH 1.2) and is rapidly dissolved within 10 minutes under the pH condition of the small intestine (near pH 6.8), it has been used as a coating agent for enteric capsules and various tablets. In addition, HPMCP has been used in a variety of applications including excipients for medicines, binders, and solubility-enhancer.

HPMCP has been conventionally prepared by a method of mixing an acetic acid solvent with hydroxypropyl methylcellulose, phthalic anhydride, and sodium acetate as a catalyst to bind only one side of phthalic acid to a cellulose molecule.

With this conventional method, Patent Document 1 (US Patent No. 3,629,237) discloses a method of mixing hydroxypropyl methylcellulose with a viscosity of 15 to 100 cps, phthalic anhydride, and sodium acetate in an acetic acid solvent and allowing reaction thereof at 80°C for 5 hours to prepare a reaction solution, followed by slurrying the reaction solution in water to prepare HPMCP particles.

HPMCP particles prepared by this method commonly have a spherical shape. Such spherical HPMCP particles have low particle penetrability by a solvent such as acetone and tend to be slowly dissolved from surfaces thereof. Accordingly, spherical HPMCP particles disadvantageously have low solubility in a solvent.

However, when the HPMCP particles are used as a raw material of a composite preparation for hard capsules or as a coating solution of a pharmaceutical preparation, a process of dissolving the same in a solvent is required as needed. Accordingly, it is necessary to improve the solubility of the HPMCP particles in a solvent.

For example, Patent Document 2 (Korean Patent Application Publication No. 10-2012-0135063) discloses that a solution containing a cellulose-based enteric polymer, inorganic particles, or other additives and a solvent can be used as a coating solution for coating drug particles, HPMCP is exemplified as the cellulose-based enteric polymer, and the coating solution includes acetone.

However, it takes a long time to completely dissolve HPMCP particles in acetone upon preparation of a coating solution due to the aforementioned dissolution tendency of HPMCP particles. In addition, when undissolved particles remain in the coating solution, the medicine may have a gritty feeling when taken orally.

Accordingly, there is a need for development of a method capable of changing conventional spherical particles into porous particles such that a solvent easily penetrates into HPMCP particles and a method of providing HPMCP particles having improved solubility.

[Related Art Documents]

[Patent Documents]

(Patent Document 1) US3629237 B
(Patent Document 2) KR1020120135063 A

[Disclosure]

[Technical Problem]

Therefore, the present invention has been made in view of the above problems, and it is one object of the present invention to provide HPMCP particles having a porous particle shape and thus being capable of providing improved solubility in a solvent.

It is another object of the present invention to provide a method of providing mechanical energy to an HPMCP reaction solution to prepare porous HPMCP particles in a process of adding the HPMCP reaction solution to water to generate HPMCP particles.

[Technical Solution]

In accordance with an aspect of the present invention, the above and other objects can be accomplished by the provision of hydroxypropyl methylcellulose phthalate (HPMCP) particles having a porous particle shape.
An average porosity of the particles may be 5 to 80%.
An average diameter ratio (a ratio of a long diameter to a short diameter) of the particles may be 1.5 to 4.
A dissolution time of the particles in acetone may be 2 minutes to 4 minutes.
A viscosity of 10 wt% mixed solution obtained by dissolving the particles in a mixed solvent (weight ratio = 1:1) of methanol and methylene chloride may be 32 to 66 cSt.
In accordance with another aspect of the present invention, there is provided a method of preparing HPMCP particles, which includes (1) preparing an HPMCP reaction solution; (2) adding water to a stirring tank including a stirring device such that the stirring device is immersed therein; (3) dropping the HPMCP reaction solution into the stirring tank; and (4) adding the dropped HPMCP reaction solution to water contained in the stirring tank, in which the stirring device is operating, to be granulated while moving along the flow of the water.
The HPMCP reaction solution of step (1) may be obtained by esterifying HPMC and phthalic anhydride in a reaction medium in the presence of a catalyst
In step (3), the HPMCP reaction solution may be dropped, in a gravity drop manner, into the stirring tank. Here, a space velocity (SV), which represents a flow rate passing through a unit volume per hour, of the HPMCP reaction solution is preferably 1.2 to 3.5/h.
In step (4), the HPMCP reaction solution may be granulated into a porous shape due to mechanical energy of the stirring device, and an agitation speed of the stirring device is preferably 500 to 2000 rpm.

[Advantageous Effects]

Since HPMCP particles according to the present invention have a porous particle shape including pores therein, a solvent can easily penetrate into the particles and the HPMCP particles have improved solubility in a solvent due to a large surface area of the particles being in contact with the solvent.
In addition, according to a method of preparing the HPMCP particles of the present invention, the HPMCP particles can be changed into porous particles by adding an HPMCP reaction solution to water contained in the stirring tank, in which the stirring device is operating, to granulate the HPMCP. Accordingly, the present invention is economical because products can be produced by changing only production process conditions without changing existing facilities.

[Description of Drawings]

FIG. 1 schematically illustrates an apparatus for preparing HPMCP particles according to one embodiment of the present invention.
FIG. 2 illustrates non-magnified photographs (naked eye) of HPMCP particles prepared according to Example 1 and Comparative Example 1 and 50x magnified SEM photographs thereof.
FIG. 3 is a set of optical microscope photographs illustrating the dissolution tendency of HPMCP particles prepared according to Example 1 in comparison with Comparative Example 1.

[Modes of the Invention]

The present invention relates to hydroxypropyl methylcellulose phthalate (HPMCP) particles having a porous particle shape and a method of preparing the same.
A porosity range of the HPMCP particles according to the present invention is not specifically limited so long as the HPMCP particles include pores into which a solvent penetrates. An average porosity of the HPMCP particles may be preferably 5 to 80%, more preferably 20 to 50%. When the average porosity is less than 5%, solubility of HPMCP particles in a solvent may be slightly improved. On the other hand, when the average porosity is greater than 80%, economic feasibility may be lowered compared to the effect.
The average porosity, which refers to a ratio of pores in particles, may be measured by a mercury intrusion method (JIS R 1655「Method of measuring pore size distribution according to mercury intrusion method of fine ceramics」, derived from a relationship between a mercury volume and the pressure when mercury enters pores).
In addition, since the HPMCP particles according to the present invention are subjected to mechanical energy in a process of being granulated into a porous particle shape, a difference between a long diameter of the HPMCP particles and a short diameter thereof is large, compared to spherical HPMCP particles prepared according to a conventional method. That is, an average diameter ratio (a ratio of a long diameter to a short diameter) of conventional spherical HPMCP particles does not exceed about 1.4, but the porous HPMCP particles prepared according to the present invention have an average diameter ratio of 1.5 or more. Preferably, the HPMCP particles according to the present invention may have an average diameter ratio of 1.5 to 4. When an average diameter ratio of the HPMCP particle is within this range, the HPMCP particles have excellent solubility in a solvent and are economical in terms of required mechanical energy. The average diameter ratio refers to an average value obtained by measuring a short diameter and long diameter of HPMCP particles photographed with an optical microscope (BX51, manufactured by Olympus) and calculating a ratio of the long diameter to the short diameter.

In addition, when acetone is added to the HPMCP particles having a porous particle shape, a dissolution time of HPMCP particles in acetone may be 2 minutes to 4 minutes. In particular, FIG. 2 illustrates SEM images of porous HPMCP particles (Example 1) according to the present invention in comparison with non-porous spherical HPMCP particles (Comparative Example 1), and FIG. 3 is a set of optical microscope photographs illustrating a dissolution degree of a particle in minutes after dropping acetone onto each particle illustrated in FIG. 2. Referring to FIG. 3, it can be confirmed that the HPMCP particles having a porous particle shape are almost completely dissolved within 3 minutes, whereas the spherical HPMCP particles maintain a particle shape thereof and are not dissolved after 3 minutes. In addition, almost half of the spherical HPMCP particles were not dissolved even after 4 minutes.

The physicochemical properties of the HPMCP particles depend upon the content and molecular weight of each substituent. Preferably, the HPMCP particles according to the present invention may have a methoxy group substitution degree of 1.6 to 2.0, a hydroxypropoxy group substitution degree of 0.2 to 0.3, and a phthalyl group substitution degree of 0.4 to 1.2, per glucose unit. In the present specification, the term "substitution degree" refers to an average number of hydroxyl groups substituted with each substituent for each glucose unit of a cellulose derivative as shown in Formula 1 below:

Formula 1

[37]

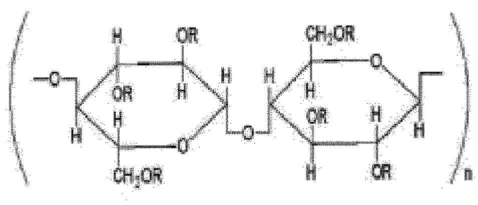

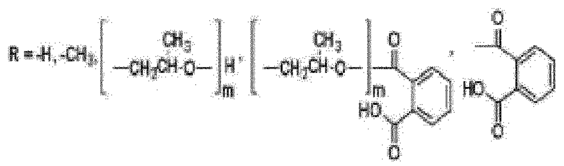

wherein m and n are an integer of 1 or more.

In addition, the viscosity of the HPMCP particles is preferably 32 to 66 centistoke (cSt). The viscosity refers to a viscosity of a 10 wt% mixed solution obtained by dissolving the HPMCP particles in a mixed solvent of methanol and methylene chloride (weight ratio = 1:1). In the present specification, the term "viscosity" refers to a viscosity measured by a capillary viscometer method according to Korean Pharmacopoeia.

Meanwhile, the present invention provides a method of preparing the HPMCP particles, the method including (1) preparing an HPMCP reaction solution; (2) adding water to a stirring tank including a stirring device such that the stirring device is immersed therein; (3) dropping the HPMCP reaction solution into the stirring tank; and (4) adding the dropped HPMCP reaction solution to water contained in the stirring tank, in which the stirring device is operating, to be granulated while moving along the flow of the water.

Hereinafter, the method of preparing the HPMCP particles according to the present invention is described in detail.

(1) Step of preparing an HPMCP reaction solution

The HPMCP reaction solution may be obtained by esterifying hydroxypropyl methylcellulose (HPMC) and

phthalic anhydride in a reaction medium in the presence of a catalyst.

Here, the catalyst, which facilitates esterification, may include an alkali metal salt of acetic acid. For example, the alkali metal salt of acetic acid may include at least one of sodium acetate and potassium acetate. The content of the catalyst may be 40 to 200 parts by weight based on 100 parts by weight of HPMC.

The reaction medium, which disperses a catalyst, HPMC, and phthalic anhydride to increase a contact area thereamong, may include at least one selected from the group consisting of acetic acid, propionic acid, and butyric acid. The reaction medium may be used in an amount of 200 to 2,000 parts by weight based on 100 parts by weight of HPMC.

The HPMC may have a methoxy group substitution degree of 1.6 to 2.0 and a hydroxypropoxy group substitution degree of 0.2 to 0.3, per glucose unit.

In the esterification, the phthalic anhydride may be used in an amount of 100 to 300 parts by weight based on 100 parts by weight of the HPMC.

The esterification may be performed at 60 to 100°C for 3 to 4 hours. When the esterification is performed within this temperature range and time range, esterification may be sufficiently performed with reasonable energy costs.

(2) Step of adding water

This step is adding water to a stirring tank including a stirring device such that the stirring device is immersed therein.

The stirring tank used in the present invention includes a stirring device therein and is generally used in the polymer synthesis field to which the present invention pertains. That is, the method of preparing the HPMCP particles according to the present invention is an economical method that does not require additional facility investment costs for implementation of the invention because it only requires changes in production process conditions and does not require changes to existing facilities.

For example, the stirring device is not specifically limited so long as it can apply mechanical energy to water contained in a stirring tank. For example, an impeller or the like may be used as the stirring device, and a vertical stirring tank in which a stirrer, to which an impeller is attached, is installed may be used as a stirring tank including the stirring device. This vertical stirring tank may make an ideal flow pattern having a high stirring effect by forming a synthetic flow of a downward flow and an axial flow.

The water may be purified water. An addition amount of the water is not limited so long as the stirring device can be sufficiently immersed. Preferably, the water may be added in a range of 12 to 20 times a total content of the reaction medium used in the esterification of step (1). When a total use amount of water is within this range, the mechanical energy of the stirring device may be sufficiently transferred to the HPMCP reaction solution through water, whereby pores may be formed in generated HPMCP particles. Accordingly, HPMCP particles having a porous particle shape may be provided. In addition, the generated HPMCP particles may be prevented from agglomerating together to form a large lump, and process efficiency may be improved due to use of an appropriate amount of water.

Since the HPMCP reaction solution is granulated while being stirred when added to water according to the method of preparing the HPMCP particles of the present invention and pores are formed inside the HPMCP particles by the mechanical energy of the stirring device in such a granulation process (see "(4) Granulation step" below), water should be supplied before the HPMCP reaction solution is supplied into the stirring tank.

(3) Step of dropping reaction solution into stirring tank

This step is dropping the HPMCP reaction solution, which has been prepared in step (1), into the stirring tank into which water has been fed according to step (2). Here, the HPMCP reaction solution may be dropped into the stirring tank in a gravity drop manner.

Here, "gravity drop manner" is a manner wherein a chamber containing a reaction solution is located at the top and the reaction solution reaches a nozzle for liquid injection by the gravity applied to the reaction solution in the chamber. FIG. 1 schematically illustrates an apparatus for preparing HPMCP particles according to one embodiment of the present invention.

Here, a space velocity (SV), which represents a flow rate passing through a unit volume per hour, of the HPMCP reaction solution falling from the chamber containing the reaction solution, is preferably 1.2 to 3.5/h. When the space velocity is less than 1.2/h, a preparation time may be extended, making the process uneconomical. On the other hand, when the space velocity is greater than 3.5/h, HPMCP particles may agglomerate to form a large lump or it may be difficult to impart porosity to particles.

(4) Step of forming particles

This step is adding the reaction solution, which has been dropped according to step (3), to water contained in

the stirring tank, in which the stirring device is operating, to be granulated while moving along the flow of the water.

In this step, the reaction solution is granulated into a porous shape by the mechanical energy of the stirring device. In particular, referring to an SEM image of HPMCP particles prepared according to Example 1 illustrated in FIG. 2, it can be confirmed that the HPMCP particles have a porous particle shape.

More particularly, referring to FIG. 1, the HPMCP reaction solution dropped, in a gravity drop manner, from the reaction solution chamber is fed into water contained in the stirring tank. At this time, water is rotating due to operation of the stirring device in the stirring tank, and the reaction solution fed into the water is granulated while rotating along the rotation direction of the water as soon as it is fed. That is, the mechanical energy of the stirring device is transferred to the reaction solution through water, and the HPMCP reaction solution is formed into porous particles due to the applied energy. The porous HPMCP particles prepared in such a manner have a large surface area for solvent/particle contact, thus having improved solubility.

On the other hand, it can be confirmed that HPMCP particles prepared according to Comparative Example 1 do not have a spherical particle shape and pores are not included therein as shown in an SEM image of FIG. 2. In a method of preparing such spherical HPMCP particles, granulation was generally performed by adding an HPMCP reaction solution to water or adding water to the reaction solution, followed by filtration and drying. However, non-porous spherical HPMCP particles prepared by such a method have a drawback of having low solubility in a solvent.

An agitation speed of the stirring device is preferably 500 to 2000 rpm. When the agitation speed is less than 500 rpm, it may be difficult to obtain porous HPMCP particles. On the other hand, when the agitation speed is greater than 2000 rpm, economic feasibility may be lowered compared to the effect.

The temperature of the reaction solution used in the step of forming particles may be 45 to 60°C. When the temperature of the reaction solution is within this range, the viscosity of the reaction solution is appropriate, whereby process efficiency increases and particles having appropriate porosity may be generated.

In addition, the temperature of the water used in the step of forming particles may be 20 to 30°C. When the temperature of the water is within this range, a reaction medium residue remaining in the reaction solution after generation of the HPMCP particles is dissolved in the water and thus may be efficiently separated from the HPMCP particles, and the generated HPMCP particles may not agglomerate with each other.

Now, the present invention will be described in more detail with reference to the following examples. These examples are provided for illustrative purposes only and should not be construed as limiting the scope and spirit of the present invention.

## Example 1

First, 100 g of HPMC (having a methoxy group substitution degree of 1.85 and a hydroxypropoxy group substitution degree of 0.27, per glucose unit), 120 g of phthalic anhydride, 380 g of acetic acid as a reaction solvent, and 50 g of sodium acetate as a catalyst were mixed. The resultant mixture was heated at 85°C for 3 hours while slowly stirring at 100 rpm, thereby being esterified. As a result, an HPMCP reaction solution was prepared.

A stirring tank provided with an impeller (T-50, manufactured by IKA) was prepared, and then 200 g of purified water was fed into the stirring tank.

Subsequently, the prepared HPMCP reaction solution was dropped into the stirring tank at a flow rate of 0.9 ℓ/s.

Subsequently, the dropped HPMCP reaction solution was fed into purified water contained in the stirring tank, in which the impeller was rotating at an agitation speed of 500 rpm, and was granulated while rotating along the flow of the purified water. As a reaction product, a slurry including HPMCP particles was prepared. Here, the HPMCP reaction solution was formed in a porous particle shape due to the mechanical energy of the impeller.

Subsequently, the slurry was washed with purified water four times, and then was dried to have a loss on drying (LOD) of less than 5% by weight using a fluid bed dryer. As a result, porous HPMCP particles were prepared.

## Examples 2 and 3

Porous HPMCP particles were prepared in the same manner as in Example 1, except that an agitation speed of an impeller included in a stirring tank, into which an HPMC reaction solution was fed, was adjusted as summarized in Table 1 below.

## Comparative Example 1

HPMCP particles were prepared in the same manner as in Example 1, except that an HPMCP reaction solution was dropped into a stirring tank, in which an impeller did not rotate, to prepare a slurry including HPMCP particles. Here, the HPMCP particles prepared according to Comparative Example 1 had a non-porous spherical particle shape.

<Evaluation methods and results>

1. Dissolution rate

360 g of HPMCP particles prepared according to each of Examples 1 to 3 and Comparative Example 1 was added to 2,640 g of acetone, and then a dissolution time thereof was measured. Results are summarized in Table 1 below.

[Table 1]

| | Agitation speed (rpm) | Dissolution time (min) | Result |
|---|---|---|---|
| Example 1 | 500 | 43 | Completely dissolved |
| Example 2 | 1000 | 40 | Completely dissolved |
| Example 3 | 2000 | 41 | Completely dissolved |
| Comparative Example 1 | 0 | 60 | Undissolved particles were present |

Referring to Table 1, it can be confirmed that the HPMCP particles prepared according to Examples 1 to 3 of the present invention exhibit a high dissolution rate in a solvent, compared to the HPMCP particles prepared according to Comparative Example 1. Here, in the HPMCP particles prepared according to Comparative Example 1, undissolved particles were present even after 60 minutes.

2. Average diameter ratio

The HPMCP particles prepared according to Example 1 and Comparative Example 1 were photographed by an optical microscope (BX51, manufactured by Olympus). Short diameters and long diameters of 50 captured HPMCP particles were measured. A diameter ratio of each particle was calculated according to Equation 1 below to find an average diameter ratio.

$$\text{Diameter ratio} = \text{long diameter of particle} / \text{short diameter of particle} \dots (1)$$

It can be confirmed that an average diameter ratio of the HPMCP particles prepared according to Example 1 is 2.4, indicating that a difference between a long diameter thereof and a short diameter thereof is large, whereas an average diameter ratio of the HPMCP particles prepared according to Comparative Example 1 is 1.3, indicating that the HPMCP particles have a near-spherical particle shape.

3. SEM analysis

A scanning electron microscope (SEM) was used to investigate the shapes of the HPMCP particles prepared according to Example 1 and Comparative Example 1. In addition, non-magnified photographs (naked eye) of the HPMCP particles and 50x magnified SEM photographs thereof were compared and shown in FIG. 2.

Referring to FIG. 2, it can be confirmed that the HPMCP particles prepared according to Example 1 have a porous structure, whereas the HPMCP particles prepared according to Comparative Example 1 have a non-porous spherical particle shape.

4. Dissolution tendency

The shapes of the HPMCP particles prepared according to each of Example 1 and Comparative Example 1 were observed for 3 minutes by an optical microscope (BX51, manufactured by Olympus) while dropping acetone thereon at a rate of 1 drop/10 sec. FIG. 3 illustrates optical microscope photographs (500x magnification) at the initial state, 1 minute, 2 minutes and 3 minutes after dropping acetone onto the respective HPMCP particles.

Referring to FIG. 3, it can be confirmed that, in the case of the HPMCP particle prepared according to Example 1, a solvent easily penetrates into the porous particle and a contact surface area between the solvent and the particle is large, whereby solubility is improved. On the other hand, it can be confirmed that, in the case of the HPMCP particle prepared according to Comparative Example 1, a solvent does not penetrate into the particle and a surface of the particle starts to slowly dissolve.

The aforementioned examples are not intended to limit the technical spirit according to the present invention

and are provided to illustrate the same. It should be understood that the scope of the present invention is defined by the following claims and the invention is to cover all technical ideas falling within the scope of the invention as defined by the claims.

## Claims

1. Hydroxypropyl methylcellulose phthalate (HPMCP) particles having a porous particle shape.

2. The HPMCP particles according to claim 1, wherein an average porosity of the particles is 5 to 80%.

3. The HPMCP particles according to claim 1, wherein an average diameter ratio (a ratio of a long diameter to a short diameter) of the particles is 1.5 to 4.

4. The HPMCP particles according to claim 1, wherein a dissolution time of the particles in acetone is 2 minutes to 4 minutes.

5. The HPMCP particles according to claim 1, wherein a 10 wt% mixed solution obtained by dissolving the particles in a mixed solvent (weight ratio = 1:1) of methanol and methylene chloride has a viscosity of 32 to 66 cSt.

6. A method of preparing hydroxypropyl methylcellulose phthalate (HPMCP) particles, the method comprising:

   (1) preparing an HPMCP reaction solution;
   (2) adding water to a stirring tank including a stirring device such that the stirring device is immersed therein;
   (3) dropping the HPMCP reaction solution into the stirring tank; and
   (4) adding the dropped HPMCP reaction solution to water contained in the stirring tank, in which the stirring device is operating, so that porous HPMCP particles are formed due to mechanical energy of the stirring device.

7. The method according to claim 6, wherein the HPMCP reaction solution of step (1) is obtained by esterifying HPMC and phthalic anhydride in a reaction medium in the presence of a catalyst.

8. The method according to claim 6, wherein, in step (3), the HPMCP reaction solution is dropped, in a gravity drop manner, into the stirring tank.

9. The method according to claim 6, wherein, in step (3), a space velocity (SV) of the HPMCP reaction solution is 1.2 to 3.5/h.

【Fig. 1】

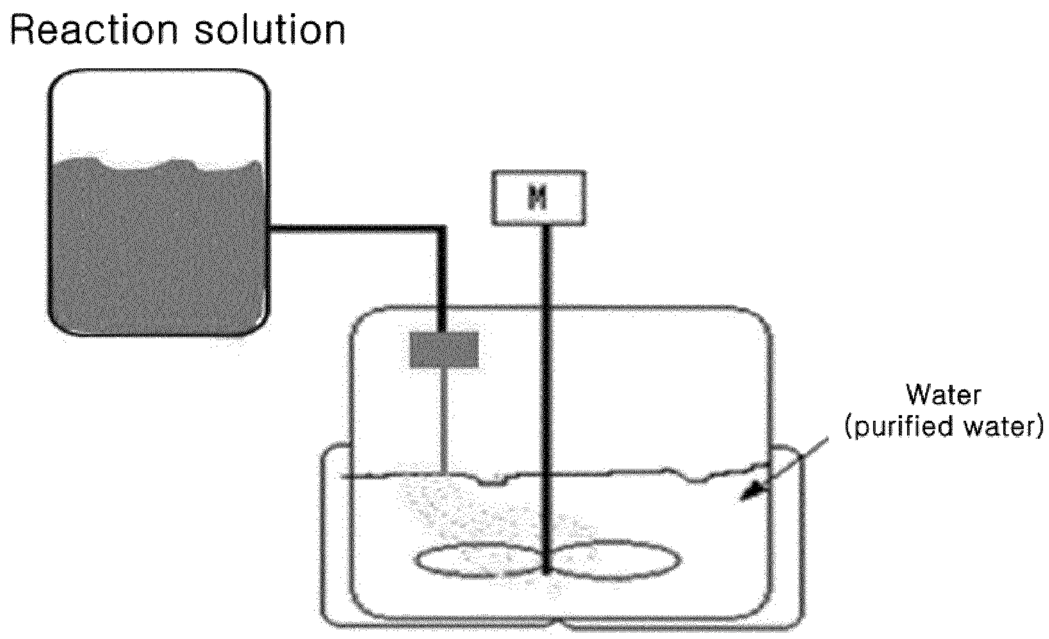

【Fig. 2】

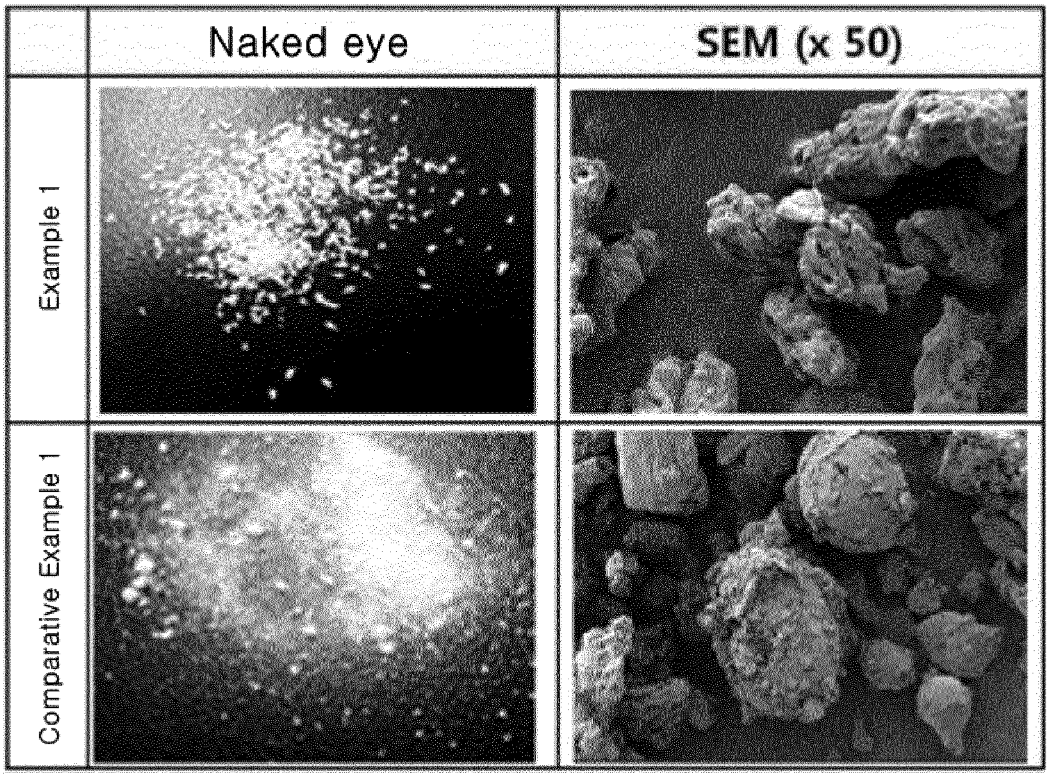

【Fig. 3】

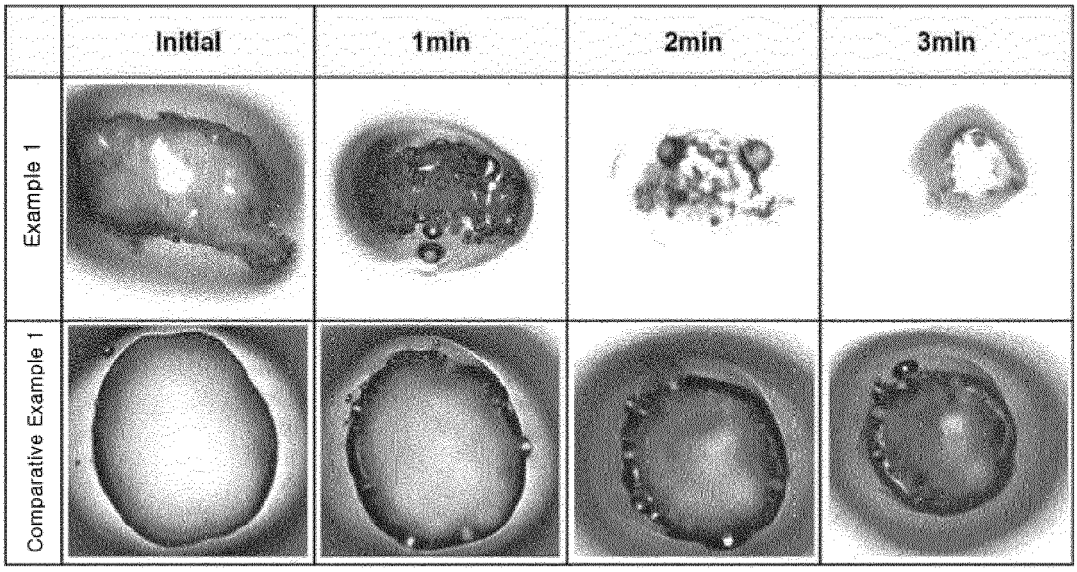

## INTERNATIONAL SEARCH REPORT

International application No.
PCT/KR2016/012621

A. CLASSIFICATION OF SUBJECT MATTER

***C08B 15/02(2006.01)i, C07C 67/62(2006.01)i, C07C 69/80(2006.01)i, B01J 19/00(2006.01)i***

According to International Patent Classification (IPC) or to both national classification and IPC

B. FIELDS SEARCHED

Minimum documentation searched (classification system followed by classification symbols)
C08B 15/02; A61K 9/14; C08B 15/08; B01D 71/12; C08B 16/00; B01D 69/02; A61K 47/04; C07C 67/62; C07C 69/80; B01J 19/00

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: porosity, hydroxypropylmethyl cellulose, HPMCP, agitating device

C. DOCUMENTS CONSIDERED TO BE RELEVANT

| Category* | Citation of document, with indication, where appropriate, of the relevant passages | Relevant to claim No. |
|---|---|---|
| X<br>Y | JP 4397146 B2 (HOSOKAWA MICRON CORP.) 13 January 2010<br>See figure 4; paragraphs [0037]-[0039]. | 1-6,8-9<br>7 |
| Y | US 3629237 A (KOYANAGI, Shunichi et al.) 21 December 1971<br>See column 3, lines 47-58; example 2. | 7 |
| A | JP 2000-229227 A (YUASA CORP.) 22 August 2000<br>See claim 1; paragraph [0015]. | 1-9 |
| A | JP 10-195103 A (CHISSO CORP.) 28 July 1998<br>See claim 6. | 1-9 |
| A | ZHAO, Xiuhua et al., "Preparation, Characterization, and Evaluation In Vivo of Ins-SiO2-HP55 (Insulin-loaded Silica Coating HP55) for Oral Delivery of Insulin", International Journal of Pharmaceutics, 15 September 2013, vol. 454, no. 1, pages 278-284<br>See abstract. | 1-9 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 FEBRUARY 2017 (16.02.2017) | **16 FEBRUARY 2017 (16.02.2017)** |

| Name and mailing address of the ISA/KR | Authorized officer |
|---|---|
| Korean Intellectual Property Office<br>Government Complex-Daejeon, 189 Seonsa-ro, Daejeon 302-701, Republic of Korea<br>Facsimile No. 82-42-472-7140 | <br>Telephone No. |

Form PCT/ISA/210 (second sheet) (January 2015)

**INTERNATIONAL SEARCH REPORT**
Information on patent family members

International application No.
**PCT/KR2016/012621**

| Patent document cited in search report | Publication date | Patent family member | Publication date |
|---|---|---|---|
| JP 4397146 B2 | 13/01/2010 | AU 2002-330390 A1 | 29/09/2003 |
| | | EP 1498116 A1 | 19/01/2005 |
| | | JP 2003-275281 A | 30/09/2003 |
| | | JP 2003-277295 A | 02/10/2003 |
| | | JP 4142318 B2 | 03/09/2008 |
| | | US 2005-0191357 A1 | 01/09/2005 |
| | | US 2008-0248119 A1 | 09/10/2008 |
| | | WO 03-077886 A1 | 25/09/2003 |
| US 3629237 A | 21/12/1971 | DE 1946170 A1 | 19/03/1970 |
| | | DE 1946170 B2 | 30/10/1975 |
| | | FR 2020529 A1 | 17/07/1970 |
| JP 2000-229227 A | 22/08/2000 | JP 4395904 B2 | 13/01/2010 |
| JP 10-195103 A | 28/07/1998 | JP 3601229 B2 | 15/12/2004 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3629237 A
- KR 1020120135063
- US 3629237 B
- KR 1020120135063 A